# EUROPEAN PATENT APPLICATION

(11) **EP 4 810 047 A1**
(43) Date of publication of application: **23.09.2026**
(21) Application number: 23958491.5
(22) Date of filing: 13.11.2023
(51) Int. Cl.: A61K 31/704, A61K 31/352, A61K 31/12, A61P 11/00

(54) **TRADITIONAL CHINESE MEDICINE COMPOSITION FOR TREATING LUNG INFLAMMATION-RELATED DISEASES AND USE THEREOF**

(71) Applicant: Xiyuan Hospital China Academy Of Chinese Medical Sciences, Beijing 100091 (CN); NICM Health Research Institute, Western Sydney University, Australia, Westmead, New South Wales 2751-1797 (AU)
(72) Inventor: LIU, Jianxun, Beijing 100091 (CN); ZHOU, Xian, Westmead, New South Wales 2751-1797 (AU); LI, Junmei, Beijing 100091 (CN); PENG, Qing, Beijing 100091 (CN); CHANG, Dennis, Westmead, New South Wales 2751-1797 (AU); REN, Junguo, Beijing 100091 (CN); LI, Chunguang, Westmead, New South Wales 2751-1797 (AU); WANG, Xinwei, Beijing 100091 (CN); MATTHEW, Shintu, Westmead, New South Wales 2751-1797 (AU)
(74) Representative: Winter, Brandl - Partnerschaft mbB
(86) International application number: PCT/CN2023/131249
(87) International publication number: WO 2025/102204

(57) **Abstract**

The present invention provides a traditional Chinese medicine composition for treating lung inflammation related diseases, comprising glycyrrhizic acid, baicalein and 6-shogaol. The traditional Chinese medicine composition of the present invention is based on the combination of multiple active ingredients of traditional Chinese medicine, which can exert synergistic effects with multiple targets and at multiple levels. The multi-ingredient combination can overcome the limited efficacy, adverse reactions and drug resistance of single-ingredient treatment, and has no toxic and side effects in the effective dose range, which can ensure the safety of clinical medication, especially for patients who are not suitable for hormones, expanding the scope of clinical application. In addition, the present invention is a composition of active ingredients of traditional Chinese medicine with clear ingredients and clear interactions among ingredients, and its efficacy and safety are higher than those of traditional Chinese medicine compound prescriptions with complex ingredients.

## Description

### TECHNICAL FIELD

The present invention relates to the field of medicinal chemistry, specifically to a traditional Chinese medicine composition for treating and/or preventing lung inflammation-related diseases and use thereof.

### BACKGROUND ART

Acute lung injury (ALI) is a severe clinical disease characterized by respiratory distress, refractory hypoxemia and non-cardiogenic pulmonary edema, which could further aggravate and develop into acute respiratory distress syndrome (ARDS). The pathological manifestations are extensive pulmonary inflammation and death or dysfunction of alveolar epithelial cells and/or pulmonary capillary endothelial cells, leading to destruction of the alveolar-capillary barrier.

ALI/ARDS has a hospital mortality rate as high as 30%-50% clinically. Especially under the background of the global outbreak of Corona Virus Disease 2019 (COVID-19), the incidence of ALI/ARDS presents sporadic outbreaks. At present, the clinical drug treatment of ALI is dominated by glucocorticoids, which achieve anti-inflammatory effects through immunosuppression. However, hormones can cause various toxic and side effects of varying degrees, including infection, gastrointestinal bleeding, electrolyte disturbance, hypertension, hyperglycemia, etc., which greatly limit clinical application. Due to the characteristics of rapid transmission and difficult prevention of respiratory infectious diseases, the development of effective and safe drugs for treating ALI/ARDS has become an urgent problem to be solved.

Modern studies have confirmed that the occurrence of ALI/ARDS is caused by pathogenic factors activating a variety of inflammatory cells and releasing a series of inflammatory mediators. These inflammatory mediators can reactivate inflammatory cells and release more inflammatory mediators or cytokines in an "autocrine" or "paracrine" manner, leading to a cascade of inflammatory responses and excessive lung injury. Lipopolysaccharide (LPS) is a component of the outer membrane of Gram-negative bacteria, which is a main pathogenic substance. After entering the body, it activates macrophages to release pro-inflammatory factors, chemotaxes and activates polymorphonuclear neutrophils which remain in the lungs, producing local inflammatory reactions and causing ALI. It damages pulmonary vascular endothelium and increases its permeability, eventually resulting in pulmonary edema and progressive gas exchange disorder. These changes are consistent with the pathological process of ALI/ARDS.

The active ingredients of traditional Chinese medicine are natural components derived from plants, which play a certain therapeutic role in the treatment of ALI. For example, the active ingredients of traditional Chinese medicine such as phenols, terpenoids, phenylpropanoids and alkaloids have the effects of reducing inflammatory reactions, suppressing oxidative stress, regulating body immunity, and improving alveolar-capillary barrier dysfunction. In addition, compared with commonly used clinical therapeutic drugs, the active ingredients of traditional Chinese medicine also have unique advantages such as low toxic effects and side effects and high safety. However, to date, there is no report in the prior art of a traditional Chinese medicine composition that can safely and effectively treat lung inflammation-related diseases caused by various factors (e.g., acute lung injury, acute respiratory distress syndrome, etc.).

### SUMMARY OF THE INVENTION

In view of the deficiencies of the prior art, one of the aims of the present invention is to provide a traditional Chinese medicine composition for treating and/or preventing lung inflammation-related diseases.

The traditional Chinese medicine composition for treating and/or preventing lung inflammation-related diseases of the present invention is realized through the following technical solutions:

A traditional Chinese medicine composition for treating lung inflammation-related diseases, comprising glycyrrhizic acid, baicalein and 6-shogaol.

According to the traditional Chinese medicine composition of the present invention, the traditional Chinese medicine composition comprises following active ingredients: glycyrrhizic acid, baicalein and 6-shogaol. Preferably, the active ingredients of the traditional Chinese medicine composition are glycyrrhizic acid, baicalein and 6-shogaol.

According to the traditional Chinese medicine composition of the present invention, the molar ratio of glycyrrhizic acid, baicalein and 6-shogaol in the traditional Chinese medicine composition is (1-16): (1-16): (1-16).

According to the traditional Chinese medicine composition of the present invention, the molar ratio of glycyrrhizic acid, baicalein and 6-shogaol in the traditional Chinese medicine composition is (4-12): (1-8): (1-12).

According to the traditional Chinese medicine composition of the present invention, the molar ratio of glycyrrhizic acid, baicalein and 6-shogaol in the traditional Chinese medicine composition is (6-10): (1-4): (1-8).

According to the traditional Chinese medicine composition of the present invention, the molar ratio of glycyrrhizic acid, baicalein and 6-shogaol in the traditional Chinese medicine composition is (7-9): (1-2): (2-6).

According to the traditional Chinese medicine composition of the present invention, the molar ratio of glycyrrhizic acid, baicalein and 6-shogaol in the traditional Chinese medicine composition is 8:1:4.

According to the traditional Chinese medicine composition of the present invention, the molar ratio of glycyrrhizic acid, baicalein and 6-shogaol in the traditional Chinese medicine composition is 1:1:1.

According to the traditional Chinese medicine composition of the present invention, the molar ratio of glycyrrhizic acid, baicalein and 6-shogaol in the traditional Chinese medicine composition is 2:1:2.

According to the traditional Chinese medicine composition of the present invention, the molar ratio of glycyrrhizic acid, baicalein and 6-shogaol in the traditional Chinese medicine composition is 8:1:8.

According to the traditional Chinese medicine composition of the present invention, the molar ratio of glycyrrhizic acid, baicalein and 6-shogaol in the traditional Chinese medicine composition is 16:1:16.

According to the traditional Chinese medicine composition of the present invention, the molar ratio of glycyrrhizic acid, baicalein and 6-shogaol in the traditional Chinese medicine composition is 1:2:2.

According to the traditional Chinese medicine composition of the present invention, the molar ratio of glycyrrhizic acid, baicalein and 6-shogaol in the traditional Chinese medicine composition is 2:2:1.

According to the traditional Chinese medicine composition of the present invention, the molar ratio of glycyrrhizic acid, baicalein and 6-shogaol in the traditional Chinese medicine composition is 8:2:16.

According to the traditional Chinese medicine composition of the present invention, the molar ratio of glycyrrhizic acid, baicalein and 6-shogaol in the traditional Chinese medicine composition is 16:2:8.

According to the traditional Chinese medicine composition of the present invention, the molar ratio of glycyrrhizic acid, baicalein and 6-shogaol in the traditional Chinese medicine composition is 1:8:8.

According to the traditional Chinese medicine composition of the present invention, the molar ratio of glycyrrhizic acid, baicalein and 6-shogaol in the traditional Chinese medicine composition is 2:8:16.

According to the traditional Chinese medicine composition of the present invention, the molar ratio of glycyrrhizic acid, baicalein and 6-shogaol in the traditional Chinese medicine composition is 8:8:1.

According to the traditional Chinese medicine composition of the present invention, the molar ratio of glycyrrhizic acid, baicalein and 6-shogaol in the traditional Chinese medicine composition is 16:8:2.

According to the traditional Chinese medicine composition of the present invention, the molar ratio of glycyrrhizic acid, baicalein and 6-shogaol in the traditional Chinese medicine composition is 1:16:16.

According to the traditional Chinese medicine composition of the present invention, the molar ratio of glycyrrhizic acid, baicalein and 6-shogaol in the traditional Chinese medicine composition is 2:16:8.

According to the traditional Chinese medicine composition of the present invention, the molar ratio of glycyrrhizic acid, baicalein and 6-shogaol in the traditional Chinese medicine composition is 8:16:2.

According to the traditional Chinese medicine composition of the present invention, the molar ratio of glycyrrhizic acid, baicalein and 6-shogaol in the traditional Chinese medicine composition is 16:16:1.

According to the traditional Chinese medicine composition of the present invention, the traditional Chinese medicine composition further comprises a pharmaceutically acceptable carrier. Generally, the "pharmaceutically acceptable carrier" refers to a medium commonly acceptable in the art for delivering biologically active substances to cells or to animals (specifically mammals), including adjuvants, excipients or vehicles, such as diluents, preservatives, fillers, flow regulators, penetration enhancers, disintegrants, wetting agents, emulsifiers, suspending agents, sweeteners, lubricants and dispersants. The choice of pharmaceutically acceptable carrier depends on the mode of administration and the nature of the dosage form.

According to the traditional Chinese medicine composition of the present invention, the traditional Chinese medicine composition is administered orally, intravenously, by local injection, by inhalation or spray, sublingually, transdermally, rectally, or by other means.

According to the traditional Chinese medicine composition of the present invention, the dosage form of the traditional Chinese medicine composition is aerosol, ointment, dripping pill, soft capsule, granule, tablet, syrup or nano-preparation, etc.

The second aim of the present invention is to provide the use of the above traditional Chinese medicine composition in the preparation of an agent or a drug for treating and/or preventing lung inflammation-related diseases.

The third aim of the present invention is to provide an agent or a drug for treating and/or preventing lung inflammation-related diseases, wherein the medicament or pharmaceutical comprises the above traditional Chinese medicine composition.

According to the agent or the drug of the present invention, the agent or the drug further comprises a pharmaceutically acceptable carrier. Generally, the "pharmaceutically acceptable carrier" refers to a medium commonly acceptable in the art for delivering biologically active substances to cells or to animals (specifically mammals), including adjuvants, excipients or vehicles, such as diluents, preservatives, fillers, flow regulators, penetration enhancers, disintegrants, wetting agents, emulsifiers, suspending agents, sweeteners, lubricants and dispersants. The choice of pharmaceutically acceptable carrier depends on the mode of administration and the nature of the dosage form.

According to the medicament or pharmaceutical of the present invention, the medicament or pharmaceutical is administered orally, intravenously, by local injection, by inhalation or spray, sublingually, transdermally, rectally, or by other means.

According to the medicament or pharmaceutical of the present invention, the dosage form of the medicament or pharmaceutical is aerosol, ointment, dripping pill, soft capsule, granule, tablet, syrup or nano-preparation, etc.

It should be noted that the carriers, administration modes and dosage forms of the traditional Chinese medicine composition of the present invention are not limited to the above contents.

The "lung inflammation-related diseases" in the present invention refer to pneumonia , acute lung injury and lung diseases pathologically consistent with pneumonia and/or acute lung injury caused by various factors. The "pneumonia and acute lung injury caused by various factors" in the present invention mean lung diseases pathologically consistent with pneumonia and/or acute lung injury caused by infectious factors (viruses or bacteria) and non-infectious factors (foreign body inhalation), but are not limited thereto.

The advantages of the present invention lie in:
1. The traditional Chinese medicine composition of the present invention is used for treating acute lung injury (ALI) caused by various causes. At present, there is a lack of clinical drugs for this disease, and the present invention can fill this gap.
2. The traditional Chinese medicine composition of the present invention is based on the combination of multiple active ingredients of traditional Chinese medicine, which can exert synergistic effects with multiple targets and at multiple levels. The multi-ingredient combination can overcome the limited efficacy, adverse reactions and drug resistance of single-ingredient treatment. The results of animal experiments show that the present invention significantly improves the pathological damage of ALI induced by LPS, has a significant inhibitory effect on pulmonary edema, and can effectively prevent clinical death caused by respiratory failure due to pulmonary edema.
3. The ingredients contained in the traditional Chinese medicine composition of the present invention and their combinations have been proved by a large number of experimental studies to have no toxic and side effects in the effective dose range, which can ensure the safety of clinical medication, especially for patients who are not suitable for hormones, expanding the scope of clinical application.
4. The present invention is a composition of active ingredients of traditional Chinese medicine, where the ingredients and their interactions are clear, and its efficacy and safety are higher than those of traditional Chinese medicine compound prescriptions with complex ingredients.

### BRIEF DESCRIPTION OF THE DRAWINGS

FIG 1 shows the leukocyte count in BALF (bronchoalveolar lavage fluid) of mice in each administration group.
FIG 2 shows the pathological histomorphology of lung tissue of mice in each administration group (HE staining, 200×).

### DETAILED DESCRIPTION

The present invention will be further described below with reference to experimental examples. The following experimental examples are illustrative rather than restrictive, and the protection scope of the present invention cannot be limited by the following experimental examples.

The information of materials used in the experimental examples of the present invention is as follows:
Glycyrrhizic acid, baicalein and 6-shogaol were all purchased from MedChemExpress (MCE), wherein the HPLC (high-performance liquid chromatography) purity of glycyrrhizic acid is ≥98.0%, batch number: HY-NO184-252139; the HPLC purity of baicalein is ≥98.84%, batch number: HY-N0196-24282; the HPLC purity of 6-shogaol is ≥99.78%, batch number: HY-14616-150779. Lipopolysaccharides (from Escherichia coli O55:B55) were purchased from Sigma, USA, batch number: 039M4004V.

RAW264.7 (mouse mononuclear macrophage leukemia cells), catalogue number: CL-0190; THP-1 (human mononuclear cell leukemia cells), catalogue number CL-0233. The culture medium for RAW264.7 cells is DMEM high-glucose medium supplemented with 10% fetal bovine serum (FBS) and 1% penicillin-streptomycin double antimicrobial (P/S); the culture medium for THP-1 cells is RPMI-1640 medium supplemented with 10% fetal bovine serum (FBS), 1% penicillin-streptomycin double antimicrobial (P/S) and 0.05 mM β-mercaptoethanol. The above cells and culture reagents were purchased from Wuhan Procell Life Technology Co., Ltd. Nitric oxide detection kit was purchased from Shanghai Beyotime Biotechnology Co., Ltd., product catalogue number: S0024; mouse TNF-α, IL-6, IL-1β ELISA detection kits were purchased from Cohesion Biosciences, UK, catalogue numbers: CK5E65C, CK7E14B, CK7E88C; human IL-6 ELISA detection kit was purchased from Cohesion Biosciences, UK, catalogue number: CEK1239. Male Balb/c mice were purchased from SPF (Beijing) Biotechnology Co., Ltd.

Hereinafter, the therapeutic and/or preventive effects of the traditional Chinese medicine composition of the present invention on pneumonia and acute lung injury are specifically illustrated with reference to experimental examples.

### Experimental Example 1: Analysis of Synergistic Effect of Each Component in the Traditional Chinese Medicine Composition

NICM Health Research Institute (NICM HRI) has reported that luteolin, glycyrrhizic acid, andrographolide, baicalein, 6-shogaol and tetrandrine have a certain effect in inhibiting LPS-induced NO release from RAW264.7 cells. The present invention further found through experiments that the combination of glycyrrhizic acid, baicalein and 6-shogaol has a certain synergistic effect. The following contents include *in vitro* experiments and prediction and verification of the interaction among the three traditional Chinese medicine components using different mathematical models.

RAW264.7 cells were seeded in a 96-well cell culture plate at 1×10⁶/rnL and cultured for 24 h. Then, four groups of drugs, namely glycyrrhizic acid (100 µM, containing 0.1% DMSO), baicalein (100 µM, containing 0.1% DMSO), 6-shogaol (100 µM, containing 0.1% DMSO) and the three-component composition (composition with a molar ratio of glycyrrhizic acid:baicalein:6-shogaol of 1:1:1, total 100 µM, containing 0.1% DMSO), were serially diluted. The cells were pretreated with the serially diluted drug solutions for 2 h, and then treated with LPS (50 ng/mL) for 24 h. The cell supernatant was aspirated, the NO release level in the supernatant was determined, and the IC₅₀ values of the four groups of drugs inhibiting NO release were calculated.

**TABLE 1: Effects of glycyrrhizic acid, baicalein and 6-shogaol at various concentrations on NO production rate in LPS-induced RAW264.7 cells**

| Concentration (µM) | NO Production Rate (%)* | | |
|---|---|---|---|
| | Glycyrrhizic Acid | Baicalein | 6-Shogaol |
| 0 | 100+0.00 | 100+0.00 | 100+0.00 |
| 0.86 | 105.02±8.02 | 90.14±7.64 | NA |
| 1.63 | 88.06+5.38 | 78.17+5.06 | 60.42+6.14 |
| 3.125 | 86.28+2.29 | 73.46±7.82 | 56.75+6.97 |
| 6.25 | 81.79+5.81 | 70.08+6.12 | 41.41+2.75 |
| 12.5 | 80.67±4.74 | 60.52±5.14 | 11.42+2.69 |
| 25 | 71.04+3.50 | 40.47+6.87 | 8.67+0.84 |
| 50 | 55.35+3.98 | 7.77+3.47 | 6.11+2.41 |
| 100 | 43.54+6.10 | 2.44+2.33 | 5.89+3.38 |

| | | | |
|---|---|---|---|
| *The NO production at a drug concentration of 0 under LPS stimulation was set as 100%, and the NO production of cells without LPS stimulation was set as 0%. All NO production results are expressed as mean ± variance. | | | |

According to the above table, the IC₅₀ values (concentration at which NO production is 50%) of glycyrrhizic acid, baicalein and 6-shogaol were calculated to be 78.78±11.93 µM, 11.30±1.32 µM and 3.23±0.42 µM, respectively.

**TABLE 2: Effect of the three-component composition at various concentrations on NO production rate in LPS-induced RAW264.7 cells**

| Concentration (µM) | NO Production Rate (%)* |
|---|---|
| 0.78 | 84.98+5.63 |
| 1.56 | 72.54±7.00 |
| 3.13 | 42.64±9.02 |
| 6.25 | 24.06±9.91 |
| 12.5 | 13.68±10.78 |
| 25 | 2.83±4.22 |
| 50 | 1.44±3.15 |
| 100 | 0.22±3.45 |

| | |
|---|---|
| *The NO production at a drug concentration of 0 under LPS stimulation was set as 100%, and the NO production of cells without LPS stimulation was set as 0%. All NO production results are expressed as mean ± variance. | |

According to the above table, the IC₅₀ value (concentration at which NO production is 50%) of the three-component composition of glycyrrhizic acid, baicalein and 6-shogaol was calculated to be 2.85±0.14 µM.

The dose-effect relationships of the three single components and the three-component composition were imported into CalcuSyn software 2.0 (Biosoft, USA), and the inhibition rate-combination index plot (Fa-CI) was generated using the Chou-Talalay mathematical model. The Fa-CI plot can reflect the relationship between the CI value and the effective level of a certain biological index (i.e., the inhibitory effect on NO). The intensity and nature of the interaction between drugs can be quantitatively judged by the value of CI: CI>1 indicates antagonism, CI=1 indicates additive effect, and CI<1 indicates synergism.

**TABLE 3: Analysis of synergistic effect of glycyrrhizic acid, baicalein and 6-shogaol**

| Administration Group | IC₅₀ (µM) | CI Value |
|---|---|---|
| Glycyrrhizic Acid | 78.78±11.93 | |
| Baicalein | 11.30±1.32 | |
| 6-Shogaol | 3.23±0.42 | |
| Three-Component Composition | 2.85+0.14 | 0.45 |

Conclusion: The experimental results are shown in TABLEs 1, 2 and 3. The IC₅₀ value of the three-component group is lower than that of each single component. When all drugs have a 50% inhibition rate on NO in the supernatant of LPS-induced RAW264.7 cells, the drug concentration required by the three-component group is lower than that required by each single component; meanwhile, the CI value of the three-component group is <1, indicating that there is a certain synergistic effect in the combination of the three components.

### Experimental Example 2: Drug Preparation

1. Preparation of test drug solutions for Experimental Examples 3 and 4

A total of 16 administration groups were set in Experimental Examples 3 and 4 of the present invention, and the mass of active ingredients of traditional Chinese medicine in the test drug solutions of each administration group was accurately weighed according to TABLE 4.

**TABLE 4: Weighed mass of each active ingredient of traditional Chinese medicine in each traditional Chinese medicine composition in Experimental Examples 3 and 4**

| Administration Group | Mass (mg) | | |
|---|---|---|---|
| | Glycyrrhizic Acid | Baicalein | 6-Shogaol |
| 1 | 25.72 | 8.45 | 8.64 |
| 2 | 51.43 | 8.45 | 17.27 |
| 3 | 205.73 | 8.45 | 69.09 |
| 4 | 411.47 | 8.45 | 138.19 |
| 5 | 25.72 | 16.89 | 17.27 |
| 6 | 51.43 | 16.89 | 8.64 |
| 7 | 205.73 | 16.89 | 138.19 |
| 8 | 411.47 | 16.89 | 69.09 |
| 9 | 25.72 | 67.56 | 69.09 |
| 10 | 51.43 | 67.56 | 138.19 |
| 11 | 205.73 | 67.56 | 8.64 |
| 12 | 411.47 | 67.56 | 17.27 |
| 13 | 25.72 | 135.12 | 138.19 |
| 14 | 51.43 | 135.12 | 69.09 |
| 15 | 205.73 | 135.12 | 17.27 |
| 16 | 411.47 | 135.12 | 8.64 |

The drugs of each group weighed according to TABLE 4 were placed in a 15 mL centrifuge tube, added with 10 mL dimethyl sulfoxide (DMSO) solvent, and then placed in an ultrasonic cleaner for sufficient ultrasonic oscillation to fully dissolve the drugs. 10 µL of the above DMSO solution with dissolved drugs was added to 9.99 mL of pre-prepared complete medium, so that the final volume of DMSO in each test drug solution accounted for 0.1% of the total drug solution volume, and the drug concentration in each test drug solution also reached the pre-set administration concentration, as shown in TABLE 5.

**TABLE 5: Molar concentrations of each active ingredient of traditional Chinese medicine in the test drug solutions of administration groups in Experimental Examples 3 and 4**

| Administration Group Drug Concentration (µM) | | | |
|---|---|---|---|
| | Glycyrrhizic Acid | Baicalein | 6-Shogaol |
| 1 | 3.125 | 3.125 | 3.125 |
| 2 | 6.25 | 3.125 | 6.25 |
| 3 | 25 | 3.125 | 25 |
| 4 | 50 | 3.125 | 50 |
| 5 | 3.125 | 6.25 | 6.25 |
| 6 | 6.25 | 6.25 | 3.125 |
| 7 | 25 | 6.25 | 50 |
| 8 | 50 | 6.25 | 25 |
| 9 | 3.125 | 25 | 25 |
| 10 | 6.25 | 25 | 50 |
| 11 | 25 | 25 | 3.125 |
| 12 | 50 | 25 | 6.25 |
| 13 | 3.125 | 50 | 50 |
| 14 | 6.25 | 50 | 25 |
| 15 | 25 | 50 | 6.25 |
| 16 | 50 | 50 | 3.125 |

### 2. Preparation of LPS

(1) Preparation of LPS in Experimental Examples 3 and 4: Weigh 1 mg LPS and dissolve it in 1 mL complete medium to prepare 1 mg/mL LPS stock solution, which is subsequently diluted to 50 ng/mL for subsequent cell experiments.
(2) Preparation of LPS in Experimental Examples 5 and 6: Weigh 4 mg LPS and dissolve it in 1 mL physiological saline to prepare 4 mg/mL LPS stock solution, which is subsequently diluted to 0.4 mg/mL for subsequent animal experiments.

### 3. Preparation of the three-component test drug solution for Experimental Examples 5 and 6

Accurately weigh 164.59 mg glycyrrhizic acid, 6.76 mg baicalein and 27.64 mg 6-shogaol, fully dissolve them in 1 mL DMSO, then add 4 mL PEG300 and mix evenly; add 0.5 mL Tween-80 to the above system, mix evenly, then add 4.5 mL physiological saline to make up to 10 mL, obtaining a 10 mL solution system (glycyrrhizic acid 20 mM, baicalein 2.5 mM, 6-shogaol 10 mM) with a molar ratio of glycyrrhizic acid:baicalein:6-shogaol of 8:1:4, for subsequent animal experiments.

### Experimental Example 3: Inhibitory Effect of the Traditional Chinese Medicine Composition on the Release of Inflammatory Mediators and Inflammatory Factors in RAW264.7 Cells

RAW264.7 cells were seeded in a 24-well plate at a density of 1×10⁵/mL and cultured for 24 h. After treating RAW264.7 cells with the test drug solutions of Groups 1-16 for 2 h respectively, complete medium containing 50 ng/mL LPS prepared in Experimental Example 2 was added and cultured for 24 h. The cell supernatant was collected. The release levels of NO, TNF-α and IL-6 in the cell supernatant were detected using nitric oxide detection kit, mouse TNF-α detection kit and mouse IL-6 detection kit respectively. The results of the average release levels of NO, TNF-α and IL-6 in RAW264.7 cells are shown in TABLE 6. The results in TABLE 6 were imported into IBM SPSS Statistics Software (4.0, USA) for analysis of variance of the three indicators one by one, and the specific results are shown in TABLEs 7 to 9.

### Experimental Results

**TABLE 6: Average release levels of inflammatory mediators and inflammatory factors in the cell supernatant of each administration group (n≥3)**

| Administration Group | NO Average Release Level (µM) | TNF-α Average Release Level (pg/mL) | IL-6 Average Release Level (pg/mL) |
|---|---|---|---|
| 1 | 97.86 | 68.24 | 61.79 |
| 2 | 75.75 | 69.97 | 82.70 |
| 3 | 47.35 | 68.65 | 59.09 |
| 4 | 27.72 | 52.17 | 57.88 |
| 5 | 48.80 | 86.82 | 70.12 |
| 6 | 70.89 | 67.32 | 75.92 |
| 7 | 18.40 | 39.63 | 49.34 |
| 8 | 17.43 | 29.06 | 50.85 |
| 9 | 54.63 | 74.86 | 64.94 |
| 10 | 33.74 | 67.82 | 71.80 |
| 11 | 62.52 | 86.73 | 62.89 |
| 12 | 49.82 | 81.36 | 65.85 |
| 13 | 38.80 | 89.84 | 68.07 |
| 14 | 41.92 | 84.09 | 78.40 |
| 15 | 24.46 | 66.68 | 71.23 |
| 16 | 16.22 | 48.44 | 73.52 |

**TABLE 7: Analysis of variance of the average NO release level in the supernatant of RAW264.7 cells in administration groups 1-16**

| Adminis tration Group | n | Group | | | | | | | | |
|---|---|---|---|---|---|---|---|---|---|---|
| | | 1 | 2 | 3 | 4 | 5 | 6 | 7 | 8 | 9 |
| 16 | 2 | 16.2 | | | | | | | | |
| | 9 | 162 | | | | | | | | |
| 8 | 2 | 17.4 | | | | | | | | |
| | 9 | 350 | | | | | | | | |
| 7 | 2 | 18.3 | | | | | | | | |
| | 9 | 961 | | | | | | | | |
| 15 | 2 | 24.4 | 24.4 | | | | | | | |
| | 9 | 592 | 592 | | | | | | | |
| 4 | 2 | 27.7 | 27.7 | 27.7 | | | | | | |
| | 9 | 194 | 194 | 194 | | | | | | |
| 10 | 2 | | 33.7 | 33.7 | 33.7 | | | | | |
| | 9 | | 447 | 447 | 447 | | | | | |
| 13 | 2 | | 33.8 | 33.8 | 33.8 | 33.8 | | | | |
| | 9 | | 002 | 002 | 002 | 002 | | | | |
| 14 | 2 | | | 41.9 | 41.9 | 41.9 | 41.9 | | | |
| | 9 | | | 197 | 197 | 197 | 197 | | | |
| 3 | 2 | | | | 47.3 | 47.3 | 47.3 | | | |
| | 9 | | | | 511 | 511 | 511 | | | |
| 5 | 2 | | | | | 48.7 | 48.7 | 48.7 | | |
| | 9 | | | | | 962 | 962 | 962 | | |
| 12 | 2 | | | | | 49.8 | 49.8 | 49.8 | | |
| | 9 | | | | | 216 | 216 | 216 | | |
| 9 | 2 | | | | | | 54.6 | 54.6 | | |
| | 9 | | | | | | 280 | 280 | | |
| 11 | 2 | | | | | | | 62.5 | 62.5 | |
| | 8 | | | | | | | 229 | 229 | |
| 6 | 2 | | | | | | | | 70.8 | |
| | 9 | | | | | | | | 921 | |
| 2 | 2 | | | | | | | | 75.7 | |
| | 7 | | | | | | | | 473 | |
| 1 | 2 | | | | | | | | | 97.85 |
| | 3 | | | | | | | | | 97 |
| Signific ance | | 0.16 | 0.05 | 0.05 | 0.06 | 0.15 | 0.09 | 0.06 | 0.06 | 1.000 |
| | | 3 | 4 | 7 | 8 | 4 | 8 | 6 | 7 | |

**TABLE 8: Analysis of variance of the average IL-6 release level in the supernatant of RAW264.7 cells in administration groups 1-16**

| Administration Group | n | Group | | |
|---|---|---|---|---|
| | | 1 | 2 | 3 |
| 8 | 8 | 50.8512 | | |
| 11 | 7 | 55.5474 | 55.5474 | |
| 4 | 9 | 57.8769 | 57.8769 | |
| 3 | 7 | 59.0907 | 59.0907 | |
| 1 | 8 | 61.7909 | 61.7909 | 61.7909 |
| 7 | 6 | 64.6711 | 64.6711 | 64.6711 |
| 9 | 7 | 64.9398 | 64.9398 | 64.9398 |
| 12 | 3 | 65.8467 | 65.8467 | 65.8467 |
| 13 | 5 | 68.0652 | 68.0652 | 68.0652 |
| 5 | 8 | 70.1184 | 70.1184 | 70.1184 |
| 15 | 7 | 71.2287 | 71.2287 | 71.2287 |
| 10 | 5 | 71.8047 | 71.8047 | 71.8047 |
| 16 | 6 | 73.5220 | 73.5220 | 73.5220 |
| 6 | 8 | | 75.9152 | 75.9152 |
| 14 | 7 | | 78.3950 | 78.3950 |
| 2 | 10 | | | 82.7004 |
| Significance | | 0.060 | 0.059 | 0.082 |

**TABLE 9: Analysis of variance of the average TNF-α release level in the supernatant of RAW264.7 cells in administration groups 1-16**

| Administration Group | n | Group | | | | |
|---|---|---|---|---|---|---|
| | | 1 | 2 | 3 | 4 | 5 |
| 8 | 3 | 29.0633 | | | | |
| 7 | 3 | 39.6333 | 39.6333 | | | |
| 16 | 3 | 48.4433 | 48.4433 | 48.4433 | | |
| 4 | 3 | | 52.1767 | 52.1767 | 52.1767 | |
| 15 | 3 | | | 66.6767 | 66.6767 | 66.6767 |
| 6 | 3 | | | 67.3133 | 67.3133 | 67.3133 |
| 10 | 3 | | | 67.8233 | 67.8233 | 67.8233 |
| 1 | 3 | | | 68.2367 | 68.2367 | 68.2367 |
| 3 | 3 | | | 68.6500 | 68.6500 | 68.6500 |
| 2 | 3 | | | 69.9700 | 69.9700 | 69.9700 |
| 9 | 3 | | | | 74.8567 | 74.8567 |
| 12 | 3 | | | | | 81.3633 |
| 14 | 3 | | | | | 84.0933 |
| 11 | 3 | | | | | 86.7267 |
| 5 | 3 | | | | | 86.8233 |
| 13 | 3 | | | | | 89.8400 |
| Significance | | 0.066 | 0.230 | 0.063 | 0.050 | 0.051 |

According to TABLE 7, the analysis of variance of NO release level in the supernatant of RAW264.7 cells shows that 5 administration groups exhibit significant inhibitory effects on NO release in terms of inhibiting the release of inflammatory mediator NO, namely: Administration Group 16, Administration Group 8, Administration Group 7, Administration Group 15 and Administration Group 4.

According to TABLE 8, in terms of inhibiting the release of inflammatory factor IL-6, Administration Group 8 can effectively reduce the release level of inflammatory factor IL-6 in the supernatant of RAW264.7 cells.

According to TABLE 9, 3 administration groups, namely Administration Group 8, Administration Group 7 and Administration Group 16, all have significant inhibitory effects on the release of inflammatory factor TNF-α.

### Experimental Example 4: Inhibitory Effect of the Traditional Chinese Medicine Composition on the Release of Inflammatory Factor IL-6 in THP-1 Cells

Human monocyte cell line THP-1 cells were cultured in RPMI-1640 medium containing 10% fetal bovine serum, 100 U/mL streptomycin and 100 µg/mL penicillin in a cell incubator at 37 °C with 5% CO₂. THP-1 cells in the logarithmic growth phase were selected and induced to M0-type macrophages using phorbol ester. On this basis, the medium was replaced, and THP-1 cells successfully induced to M0-type macrophages were treated with drugs of Administration Groups 1-16 for 0.5 h, followed by treatment with LPS for 24 h. The cell supernatant was collected, and the release level of IL-6 in the cell supernatant was detected using human IL-6 detection kit. The experimental results are shown in TABLE 10. The results in TABLE 10 were imported into IBM SPSS Statistics Software (4.0, USA) for Analysis of variance, and TABLE 11 was obtained.

### Experimental Results

**TABLE 10: Average release level of inflammatory factor IL-6 in the cell supernatant of each group (n≥3)**

| Group | IL-6 Average Release Level (pg/mL) | Replicates after Normality Test |
|---|---|---|
| 1 | 18.21 | 4 |
| 2 | 18.04 | 5 |
| 3 | 18.21 | 4 |
| 4 | 74.65 | 4 |
| 5 | 14.40 | 3 |
| 6 | 5.44 | 5 |
| 7 | 8.40 | 5 |
| 8 | 8.58 | 4 |
| 9 | 8.72 | 5 |
| 10 | 13.59 | 5 |
| 11 | 9.44 | 5 |
| 12 | 98.10 | 3 |
| 13 | 36.31 | 4 |
| 14 | 12.00 | 5 |
| 15 | 98.39 | 3 |
| 16 | 20.11 | 4 |

**TABLE 11: Analysis of variance of the average IL-6 release level in the supernatant of human monocyte cell line THP-1 induced to differentiate into M0 macrophages in each group**

| Administration Group | n | Group | | | |
|---|---|---|---|---|---|
| | | 1 | 2 | 3 | 4 |
| 6 | 5 | 5.4340 | | | |
| 7 | 5 | 8.4020 | | | |
| 8 | 4 | 8.5825 | | | |
| 9 | 5 | 8.7220 | | | |
| 11 | 5 | 9.4420 | | | |
| 14 | 5 | 11.9960 | | | |
| 10 | 5 | 13.5920 | | | |
| 5 | 3 | 14.4033 | | | |
| 2 | 5 | 18.0380 | | | |
| 1 | 4 | 18.2150 | | | |
| 3 | 4 | 18.2150 | | | |
| 16 | 4 | 20.1100 | | | |
| 13 | 4 | | 36.3100 | | |
| 4 | 4 | | | 74.6500 | |
| 12 | 3 | | | | 98.1000 |
| 15 | 3 | | | | 98.3933 |
| Significance | | 0.127 | 1.000 | 1.000 | 0.970 |

| | | | | | |
|---|---|---|---|---|---|
| Note: The inhibitory effect on IL-6 release in THP-1 supernatant decreases from top to bottom. | | | | | |

The results in TABLE 11 show that 12 administration groups can effectively inhibit the release of IL-6 from human monocyte cell line THP-1 induced to differentiate into M0 macrophages, namely: Administration Group 6, Administration Group 7, Administration Group 8, Administration Group 9, Administration Group 11, Administration Group 14, Administration Group 10, Administration Group 5, Administration Group 2, Administration Group 1, Administration Group 3 and Administration Group 16.

Conclusion: According to Experimental Examples 3 and 4, the traditional Chinese medicine composition of the present invention can effectively inhibit the release of inflammatory factors and inflammatory mediators from RAW264.7 cells and human monocyte cell line THP-1, and has good potential for the treatment of acute lung injury.

### Experimental Example 5: Improvement Effect of the Traditional Chinese Medicine Composition on Mouse Acute Lung Injury Model

Establishment of mouse acute lung injury model: Mice were anesthetized with isoflurane, and LPS (1 mg/kg) was instilled into one side of the nasal cavity. After instillation, the mice were stood upright quickly, and returned to the original feeding cage after waking up to complete modeling.

The experiment was divided into control group (without induction of acute lung injury, intragastric administration of equal volume of normal saline), model group (with induction of acute lung injury, intragastric administration of equal volume of normal saline), three-component group (with induction of acute lung injury, intragastric administration of the three-component test drug solution prepared in Item 3 of Experimental Example 2, 398 mg/kg), and dexamethasone group (with induction of acute lung injury, intraperitoneal injection at a dose of 5 mg/kg). All drugs were administered 0.5 h after establishing the mouse ALI model with LPS.

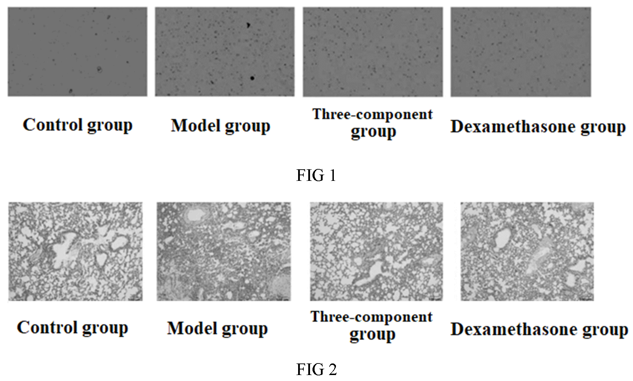

24 h after model establishment, mice were anesthetized with pentobarbital sodium and sacrificed by abdominal aorta bloodletting. Lung tissues were dissected and weighed, and the lung coefficient (lung tissue weight/mouse body weight) was calculated; the right upper lobe of the mouse lung was fixed in formalin, and pathological sections were made and stained with HE to observe pathological changes; the middle and lower lobes of the right lung of the mouse were weighed (wet weight), placed in a 60 °C oven to dry to constant weight, and weighed (dry weight), and the wet-to-dry weight ratio (wet weight/dry weight, W/D) was calculated. The experimental results are shown in TABLE 12 and Figure 1. The right bronchus of the mouse was ligated, the left lobe of the lung was taken, and bronchoalveolar lavage fluid (BALF) was recovered after lavage with 0.8 mL PBS for three times. The total number of leukocytes and total protein concentration were determined using an automatic cell counter. The experimental results are shown in TABLE 12 and Figure 1.

### Experimental Results

Compared with the model group, the three-component group can significantly reduce the lung index and wet-to-dry weight ratio of ALI mice (P<0.05), and at the same time, the three-component group can also effectively reduce the number of leukocytes and protein concentration in BALF of ALI mice (P<0.01).

Conclusion: The three-component group can effectively improve the injury of lung epithelium and vascular endothelium in ALI mice induced by LPS, effectively reduce the increase of lung tissue permeability, thereby alleviating pulmonary edema and the leakage of leukocytes and proteins.

**TABLE 12: Effects of drugs in each group on relevant indicators of ALI mice induced by LPS**

| Group | Dose (mg/kg) | Lung Index (BW) | Wet-to-Dry Ratio (W/D) | Weight BALF Leukocytes (×10⁵ cells/mL) | BALF Protein Concentration (mg/mL) |
|---|---|---|---|---|---|
| Control | | 0.619+0.038 | 4.301±0.194 | 3.689±0.887 | 0.531+0.121 |
| Model group | | 0.917+0.062## | 4.700±0.176## | 29.810±7.892## | 1.267+0.247## |
| Three-Component group | 398 | 0.869+0.040* | 4.488+0.278* | 20.420±5.364** | 0.872+0.119** |
| Dexamethasone group | 5 | 0.816+0.089** | 4.701±0.221 | 18.760±7.468** | 0.814+0.183** |

| | | | | | |
|---|---|---|---|---|---|
| Note: #P<0.05, ##P<0.01 compared with the control group; *P<0.05, **P<0.01 compared with the model group. | | | | | |

### Experimental Example 6: Improvement Effect of the Traditional Chinese Medicine Composition on Lung Inflammation in Mouse Acute Lung Injury Model

The levels of inflammatory factors IL-1β, IL-6 and TNF-α in BALF were determined in strict accordance with the instructions of the inflammatory factor detection kit.

The right upper lobe of the mouse lung was fixed with tissue fixative for 72 hours, and HE-stained sections were prepared by paraffin embedding, sectioning, dehydration and staining. Lung tissue lesions were observed under a microscope. The experimental results are shown in TABLE 13 and Figure 2.

### Experimental Results

Compared with the model group, the three-component group can significantly reduce the levels of IL-1β, IL-6 and TNF-α in BALF of ALI mice induced by LPS (P<0.01 or P<0.05); it can be observed from the pathological sections of lung tissue that compared with the model group, the three-component group significantly improves the infiltration of neutrophils and monocytes, and the pathological score of lung tissue is significantly improved compared with the model group (P<0.05).

Conclusion: The three-component group can significantly reduce the levels of inflammatory factors IL-1β, IL-6 and TNF-α in BALF, suggesting that the three-component group can effectively inhibit the infiltration and activation of inflammatory cells in lung tissue and the release of inflammatory factors to suppress cytokine storm, thereby improving ALI in mice induced by LPS.

**TABLE 13: Effects of drugs in each group on inflammatory factors in BALF of ALI mice induced by LPS**

| Group | Dose (mg/kg) | IL-1β (pg/mL) | TNF-α (pg/mL) | IL-6 (pg/mL) | Pathological Score |
|---|---|---|---|---|---|
| Control | | 12.37±7.473 | 316.960±13.920 | 15.04±7.14 | 3.157±1.25 |
| Model | | 178.67+64.787## | 533.450±173.866## | 1667.60+546.82## | 10.40+3.06 |
| Three-Component | 398 | 134.349±30.781** | 394.369±145.653* | 1167.97±403.64* | 6.70±2.50* |
| Dexamethasone | 5 | 186.475±76.810* | 345.080±239.594** | 819.19+506.63** | 6.70+2.50* |

| | | | | | |
|---|---|---|---|---|---|
| Note: #P<0.05, ##P<0.01 compared with the control group; *P<0.05, **P<0.01 compared with the model group. | | | | | |

The above are only preferred embodiments of the present invention. It should be pointed out that for those of ordinary skill in the art, several improvements and supplements can be made without departing from the method of the present invention, such as changing the ratio of the three active ingredients of traditional Chinese medicine, and these improvements and supplements shall also fall within the protection scope of the present invention.

## Claims

1. A traditional Chinese medicine composition for treating lung inflammation-related diseases, comprising glycyrrhizic acid, baicalein and 6-shogaol.

2. The traditional Chinese medicine composition according to claim 1, wherein a molar ratio of glycyrrhizic acid, baicalein and 6-shogaol is (1-16): (1-16): (1-16).

3. The traditional Chinese medicine composition according to claim 1 or 2, wherein the molar ratio of glycyrrhizic acid, baicalein and 6-shogaol is (4-12): (1-8): (1-12).

4. The traditional Chinese medicine composition according to claim 1 or 2, wherein the molar ratio of glycyrrhizic acid, baicalein and 6-shogaol is (6-10): (1-4): (1-8).

5. The traditional Chinese medicine composition according to claim 1 or 2, wherein the molar ratio of glycyrrhizic acid, baicalein and 6-shogaol is (7-9): (1-2): (2-6).

6. The traditional Chinese medicine composition according to claim 1 or 2, wherein
the molar ratio of glycyrrhizic acid, baicalein and 6-shogaol is 8:1:4; or
the molar ratio of glycyrrhizic acid, baicalein and 6-shogaol is 1:1:1; or
the molar ratio of glycyrrhizic acid, baicalein and 6-shogaol is 2:1:2; or
the molar ratio of glycyrrhizic acid, baicalein and 6-shogaol is 8:1:8; or
the molar ratio of glycyrrhizic acid, baicalein and 6-shogaol is 16:1:16; or
the molar ratio of glycyrrhizic acid, baicalein and 6-shogaol is 1:2:2; or
the molar ratio of glycyrrhizic acid, baicalein and 6-shogaol is 2:2:1; or
the molar ratio of glycyrrhizic acid, baicalein and 6-shogaol is 8:2:16; or
the molar ratio of glycyrrhizic acid, baicalein and 6-shogaol is 16:2:8; or
the molar ratio of glycyrrhizic acid, baicalein and 6-shogaol is 1:8:8; or
the molar ratio of glycyrrhizic acid, baicalein and 6-shogaol is 2:8:16; or
the molar ratio of glycyrrhizic acid, baicalein and 6-shogaol is 8:8: 1; or
the molar ratio of glycyrrhizic acid, baicalein and 6-shogaol is 16:8:2; or
the molar ratio of glycyrrhizic acid, baicalein and 6-shogaol is 1:16:16; or
the molar ratio of glycyrrhizic acid, baicalein and 6-shogaol is 2:16:8; or
the molar ratio of glycyrrhizic acid, baicalein and 6-shogaol is 8:16:2; or
the molar ratio of glycyrrhizic acid, baicalein and 6-shogaol is 16:16:1.

7. The traditional Chinese medicine composition according to claim 1 or 2, further comprising a pharmaceutically acceptable carrier.

8. The traditional Chinese medicine composition according to claim 1 or 2, which is administered orally, intravenously, by local injection, by inhalation or spray, sublingually, transdermally or rectally.

9. The traditional Chinese medicine composition according to claim 1 or 2, wherein a dosage form is aerosol, ointment, dripping pill, soft capsule, granule, tablet, syrup or nano-preparation.

10. Use of the traditional Chinese medicine composition according to any one of claims 1-9 in the preparation of a drug or agent for treating and/or preventing lung inflammation-related diseases.

11. A drug or agent for treating and/or preventing lung inflammation-related diseases, comprising the traditional Chinese medicine composition according to any one of claims 1-9.
